# EUROPÄISCHE PATENTSCHRIFT

(11) **EP 1 960 520 B1**
(45) Veröffentlichungstag und Bekanntmachung des Hinweises auf die Patenterteilung: **12.02.2014**
(21) Anmeldenummer: 06819803.5
(22) Anmeldetag: 28.11.2006
(51) Int. Cl.: C12N 15/10

(54) **VERFAHREN ZUR ISOLIERUNG VON NUKLEINSÄUREN AUS BELIEBIGEN AUSGANGSMATERIALIEN**
METHOD OF ISOLATING NUCLEIC ACIDS FROM ANY STARTING MATERIAL
PROCÉDÉ D'ISOLATION D'ACIDES NUCLÉIQUES À PARTIR DE MATÉRIAUX DE DÉPART QUELCONQUES

(30) Priorität: 28.11.2005 DE 102005057334
(43) Veröffentlichungstag der Anmeldung: 27.08.2008
(73) Patentinhaber: AJ Innuscreen GmbH, 13125 Berlin (DE)
(72) Erfinder: TIMO, Hillebrand, 15366 Hoenow (DE)
(74) Vertreter: Wehlan, Helmut
(86) Internationale Anmeldenummer: PCT/EP2006/068980
(87) Internationale Veröffentlichungsnummer: WO 2007/060248

(56) Entgegenhaltungen:
- EP-A2- 0 818 461
- WO-A-95/34569
- WO-A-2004/055207
- WO-A-2005/007895
- WO-A-2006/052680
- WO-A1-91/07422
- DE-A1- 4 321 904
- US-A1- 2005 164 260
- HOURFAR M K ET AL: "High-throughput purification of viral RNA based on novel aqueous chemistry for nucleic acid isolation" CLINICAL CHEMISTRY, AMERICAN ASSOCIATION FOR CLINICAL CHEMISTRY, WASHINGTON, DC, US, Bd. 51, Nr. 7, Juli 2005 (2005-07), Seiten 1217-1222, XP002403248 ISSN: 0009-9147
- INGRAM L O: "MECHANISM OF LYSIS OF ESCHERICHIA COLI BY ETHANOL AND OTHER CHAOTROPIC AGENTS" JOURNAL OF BACTERIOLOGY, WASHINGTON, DC, US, Bd. 146, Nr. 1, April 1981 (1981-04), Seiten 331-336, XP008012676 ISSN: 0021-9193

## Beschreibung

Gegenstand der Erfindung ist ein universelles und stark vereinfachtes Verfahren zur Isolierung von Nukleinsäuren aus Nukleinsäuren enthaltenden unterschiedlichsten Ausgangsmaterialien, welches sowohl eine sehr hohe Qualität der zu isolierenden Nukleinsäuren garantiert als auch die Isolierung quantitativer Ausbeuten ermöglicht.

Unter klassischen Bedingungen erfolgt die Isolierung von DNA aus Zellen und Geweben dadurch, dass die Nukleinsäuren enthaltenden Ausgangsmaterialien unter stark denaturierenden und reduzierenden Bedingungen, teilweise auch unter Verwendung von proteinabbauenden Enzymen aufgeschlossen, die austretenden Nukleinsäurefraktionen über Phenol-/Chloroform-Extraktionsschritte gereinigt und die Nukleinsäuren mittels Dialyse oder Ethanolpräzipitation aus der wässrigen Phase gewonnen werden (Sambrook, J., Fritsch, E.F. und Maniatis, T., 1989, CSH, "Molecular Cloning").

Diese "klassischen Verfahren" zur Isolierung von Nukleinsäuren aus Zellen und besonders aus Geweben sind sehr zeitaufwendig (teilweise länger als 48 h ), erfordern einen erheblichen apparativen Aufwand und sind darüber hinaus auch nicht unter Feldbedingungen realisierbar. Außerdem sind solche Methoden auf Grund der verwendeten Chemikalien wie Phenol und Chloroform in einem nicht geringen Maße gesundheitsgefährdend.

Verschiedene alternative Verfahren zur Isolierung von Nukleinsäuren aus unterschiedlichen biologischen Ausgangsmaterialien ermöglichen die aufwendige und gesundheitsschädigende Phenol-/Chloroform-Extraktion von Nukleinsäuren zu umgehen sowie eine Reduzierung der zeitlichen Aufwendungen zu erreichen.

Alle diese Verfahren basieren auf einer von Vogelstein und Gillespie (Proc. Natl. Acad. Sci. USA, 1979, 76, 615-619) entwickelten und erstmals beschriebenen Methode zur präparativen und analytischen Reinigung von DNA-Fragmenten aus Agarosegelen. Die Methode kombiniert die Auflösung der die zu isolierende DNA- Bande enthaltende Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2]; 200mM NaCl; 2 mM EDTA; 50% v/v Ethanol) gewaschen und danach von den Trägerpartikeln abgelöst.

Diese Methode erfuhr bis heute eine Reihe von Modifikationen und wird zum gegenwärtigen Zeitpunkt für unterschiedliche Verfahren der Extraktion und Reinigung von Nukleinsäuren aus unterschiedlichen Herkünften angewendet (Marko, M.A., Chipperfield, R. und Birnboim, H.G., 1982, Anal. Biochem., 121, 382-387).

Darüber hinaus existieren heute weltweit auch eine Vielzahl von Reagenziensystemen, vor allem zur Reinigung von DNA-Fragmenten aus Agarosegelen und für die Isolierung von Plasmid DNA aus bakteriellen Lysaten, aber auch für die Isolierung von längerkettigen Nukleinsäuren (genomische DNA, zelluläre Gesamt-RNA) aus Blut, Geweben oder auch Zellkulturen .

Alle diese kommerziell verfügbaren Kits basieren auf dem hinlänglich bekannten Prinzip der Bindung von Nukleinsäuren an mineralische Träger unter Anwesenheit von Lösungen unterschiedlicher chaotroper Salze und verwenden als Trägermaterialien Suspensionen feingemahlener Glaspulver (z.B. Glasmilk , BIO 101, La Jolla, CA), Diatomenerden (Fa.Sigma) oder auch Silicagele. (Diagen, DE 41 39 664 A1).

Ein für eine Vielzahl unterschiedlicher Anwendungen praktikables Verfahren zur Isolierung von Nukleinsäuren ist in US 5,234,809 (Boom) dargestellt. Dort ist ein Verfahren zur Isolierung von Nukleinsäuren aus nukleinsäurehaltigen Ausgangsmaterialien durch die Inkubation des Ausgangsmaterials mit einem chaotropen Puffer und einer DNA-bindenden festen Phase beschrieben. Die chaotropen Puffer realisieren sowohl die Lyse des Ausgangsmaterials als auch die Bindung der Nukleinsäuren an die feste Phase. Das Verfahren ist gut geeignet, um Nukleinsäuren aus kleinen Probenmengen zu isolieren und findet speziell im Bereich der Isolierung viraler Nukleinsäuren seine praktische Anwendung.

Spezifische Modifikationen dieser Verfahren betreffen den Einsatz von neuartigen Trägermaterialien, welche für bestimmte Fragestellungen applikative Vorteile zeigen (WO-A 95/34569).

In neueren Patentschriften wird offenbart, dass für die Adsorption von Nukleinsäuren an die dem Fachmann bekannten und eingesetzten silikatischen Materialien auch sogenannte antichaotrope Salze als Bestandteil von Lyse/Bindungspuffer-Systemen sehr effizient und erfolgreich eingesetzt werden können (EP 1135479). Vorteil dieses Verfahrens ist, dass durch die Umgehung der Verwendung von chaotropen Salzen eine deutlich geringere gesundheitliche Gefährdung von den Extraktionssystemen ausgeht. Allerdings werden für eine effiziente Isolierung von Nukleinsäuren aus einer komplexen biologischen Probe insbesondere in Hinblick auf eine möglichst ausbeutenstarke Nukleinsäure-Gewinnung im Lysepuffer wiederum hohe Salzkonzentrationen (> 1.5 M) benötigt. So offenbart die Patentschrift, dass die Verwendung findenden Lysepuffer, Salzkonzentrationen zwischen 1,5 M - 3 M enthalten.

In der Patentschrift DE 4321904 wird ein Verfahren beschrieben, dass bei Kombination von chaotropen Hochsalzpuffer in mit alkoholischen Komponenten eine effiziente Isolierung von Nukleinsäuren möglich ist. Die in der Patentschrift ausgewiesenen Lysepuffer enthalten dabei immer Salzkonzentrationen von 4 M - 8 M, insbesondere werden als Salze Guanidinhydrochlorid, Guanidinthiocyanat oder Kaliumjodid eingesetzt. Es ist bekannt, dass diese Salze eine Lyse des Ausgangsmaterials sowie eine potente Inaktivierung nukleolytischer Enzyme realisieren. Nach Lyse des Ausgangsmaterials erfolgt dann die Zugabe eines Alkohols. Die Patentschrift offenbart, dass die Zugabe der alkoholischen Komponenten zum Hochsalz-Lysepuffer eine besonders gute Effizienz der Anbindung der Nukleinsäuren an die eingesetzten silikatischen Filtermaterialien vermittelt. Nachteil der Verwendung von Lysepuffern mit hohen Ionenstärken chaotroper Salze sind aber immer der nur limitierte und auch ineffiziente Einsatz von zusätzlichen proteolytischen Enzymen für einen effizienten Aufschluss komplexer biologischer Proben, die diese Enzyme selbst durch die proteindenaturierenden Wirkung der chaotropen Puffer geschädigt werden. Darüber hinaus sind nachfolgend extensive Waschschritte zur Entfernung der hohen Salzkonzentrationen vom eingesetzten Adsorptionsmaterial notwendig. Dem Fachmann ist bekannt, das chaotrope Salze eine stark inhibitorische Wirkung auf eine Vielzahl von downstream-Applikationen ausüben.

Die Analyse des Standes der Technik verdeutlicht eindrucksvoll, dass eine Vielzahl von Möglichkeiten existiert, Nukleinsäuren an feste Trägermaterialien, insbesondere mineralische Trägermaterialien auf Siliziumbasis, zu binden, nachfolgend zu waschen und die Nukleinsäuren vom Trägermaterial wieder abzulösen. Es wird dabei sehr deutlich, dass für die Isolierung von Nukleinsäuren aus komplexen biologischen Proben sowohl sog. chaotrope Salze oder sog. antichaotrope Salze eingesetzt werden.

In der Druckschrift WO 2005/007895 A1 wird ein Verfahren zur Aufreinigung von Nukleinsäuren in einem Festphasenassay beschrieben. Offenbart wird ein Verfahren und Kits zur Bereitstellung einer Nukleinsäure und deren Bindung an eine Festphase, umfassend eine Proteaselösung (Lysepuffer), die außerdem ein erstes Chaotrop und ein Detergenz enthalten kann. Die auf die Festphase aufzubringende Bindungslösung erhält man durch Zugabe eines zweiten Chaotrops.

Das Dokument WO 91/07422 A1 offenbart ein Lysis-Reagenz mit einem Lysepuffer, der 0,5-5 M des Chaotrops Guanidinium-HCl enthält, außerdem Proteinase K sowie Detergenzien wie Triton X. Die damit lysierte Probe wird auf eine Festphase aufgebracht, die zuvor mit einer Lösung geringer Ionenstärke präpariert wurde. Nach der Bindung folgen Wasch- und Elutionsschritte. Die Probe im Lysepuffer wird bereits vor dem Säulenauftrag mit einem Bindungspuffer Ionenstärke verdünnt.

Die Vorteile der Verwendung chaotroper Salze für Prozesse zur Isolierung und Aufreinigung von Nukleinsäuren liegen darin begründet, dass diese Salze und daraus abgeleiteten Puffer eine effiziente Denaturierung von Proteinen bewirken. Dies ermöglicht die Isolierung von Nukleinsäuren auch aus komplexen biologischen Proben ggf. ohne die Verwendung proteolytischer Enzyme. Ein weiterer Vorteil besteht darin, dass chaotrope Salze als Bestandteile von Lysepuffern insbesondere bei der Isolierung von RNA auch eine potente Inaktivierung von RNasen bewirken. Nachteilig ist aber, dass die Verwendung von DNA- und RNA- Extraktionsverfahren auf der Basis chaotroper Salze immer an hohe Ionenstärken gebunden ist. In geringen Konzentrationen ist ein effizientes Anbinden von Nukleinsäuren an bisher Verwendung fmdende chromatographische Materialien nicht möglich. Dies hat u.a. zur Folge, dass dem Fachmann bekannte Waschschritte sehr aufwendig sind, darin begründet, die hohen Salzkonzentrationen von der final zu isolierenden DNA oder RNA abzutrennen. Es ist auch bekannt, das chaotrope Komponenten eine starke inhibitorische Wirkung auf eine Vielzahl von downstream- Applikationen ausüben. Darüber hinaus sind chaotrope Salze gesundheitsgefährdend und letztlich auch sehr kostenintensiv.

Vorteile des Einsatzes von sog. antichaotropen Salzen oder nichtchaotropen Salzen (darunter wird in den zitierten Patentschriften die Gruppe von Salzen verstanden, welche in der Hofmeister-Serie am anderen Ende der Reihe in bezug auf die chaotropen Salze stehen) besteht darin, dass auch aus diesen Salzen abgeleitete Pufferformulierungen fiir die Isolierung von Nukleinsäuren eingesetzt werden können. Da es sich bei diesen Salzen aber um sog. proteinstabilisierende Salze handelt, realisiert sich der Aufschluss komplexer biologischer Proben immer nur in Gegenwart proteolytischer Enzyme. Die Effizienz von Lyseprozessen ist dabei i.d.R. immer schlechter als bei der Verwendung von chaotropen Salzen als Bestandteile von Lysepuffern. Darüber hinaus ist auch nachteilig, dass insbesondere bei der Isolierung von RNA aus komplexen biologischen Proben keine Inaktivierung von RNasen erfolgt. Dies hat zur Folge, dass eine effiziente Isolierung von RNA mittels nichtchaotroper Puffersysteme nicht möglich ist. Es zeigt sich bei der Analyse des bisherigen Standes der Technik, dass auch für antichaotrope Verfahren zur Isolierung von Nukleinsäuren Ionenstärken von > 1M eingesetzt werden müssen, um eine effiziente und quantitative Isolierung von Nukleinsäuren zu erzielen.

Der Erfindung lag deshalb die Aufgabe zu Grunde, einen Lsye- / Bindungspuffer bereitzustellen, der die im Stand der Technik genannten Nachteile beseitigt.

Die,Aufgabe wurde mit den Merkmalen der Patentansprüche gelöst.

Die erfindungsgemäßen Formulierungen zur Isolierung von Nukleinsäuren aus Nukleinsäuren enthaltenden Materialien, umfassen
- mindestens einen Puffer mit chaotropen Bestandteilen
- mindestens ein proteolytisches Enzym
- mindestens einen Puffer mit nicht-chaotropen Bestandteilen
- mindestens einen Alkohol
- an sich bekannte Detergenzien.

Gemäß einer bevorzugten Ausführungsform der Erfindung ist die Konzentration des Puffers mit chaotropen Bestandteilen kleiner als 100mM und die Konzentration des Puffers mit nicht-chaotropen Bestandteilen kleiner als 1M, vorzugsweise kleiner als 500mM.

Die alkoholische Komponente besteht aus wasserlöslichen Alkoholen, wie Methanol, Ethanol, Propanol, Isopropanol, Ethylenglycol, Polyethylenglycol oder Gycerin, wobei der Anteil der alkoholischen Komponente 20 bis 80%, vorzugsweise 45 bis 55 % beträgt.

Als Detergenzkomponente kann eine der Substanzen Polyvinylpyrrolidin, CTAB, TritonX-100, N-Lauryl-Sarkosin, Natriumcitrat, DDT oder Tween 20 eingesetzt werden.

Als feste Phase können beliebige Trägermaterialien verwendet werden, sowohl bekannte negative funktionale Oberflächen als auch positiv geladene Nylon-Membranen, Polysulfon-Membranen, Polyethersulfon-Membranen PVDF-Membranen, Membranen aus Acrylpolymeren, Ionenaustauscher-Membranen, Polyethylenfritten oder einfache Filterpapiere sowie magnetische Eisenoxydpartikel, Silicatpartikel.

Das erfindungsgemäße Verfahren zur Isolierung von Nukleinsäuren aus Nukleinsäuren enthaltenden Materialien unter Verwendung von Formulierungen nach einem der Ansprüche 1 bis 9, umfasst folgende Schritte:
- Lyse des Ausgangsmaterials

Die erfindungsgemäßen Formulierungen zur Isolierung von Nukleinsäuren aus Nukleinsäuren enthaltenden Materialien, umfassen
➢ mindestens einen Lysepuffer mit chaotropen Salzkomponenten, der zusätzlich mindestens ein proteolytisches Enzym und mindestens ein Detergenz enthält und
➢ mindestens einen Bindungspuffer mit nicht-chaotropen Salzkomponenten, der zusätzlich mindestens einen Alkohol und mindestens ein Detergenz enthält,
wobei die Konzentration der chaotropen Salzkomponenten des Lysepuffers kleiner als 100mM und die Konzentration der nicht-chaotropen Salzkomponenten des Bindungspuffers kleiner als 1M ist.

Gemäß einer bevorzugten Ausführungsform der Erfindung ist die Konzentration des Puffers mit nicht-chaotropen Bestandteilen kleiner als 500mM.

Das erfindungsgemäße Verfahren zur Isolierung von Nukleinsäuren aus Nukleinsäuren enthaltenden Materialien unter Verwendung von Formulierungen nach einem der Ansprüche 1 bis 9, umfasst folgende Schritte:
➢ Lyse des Ausgangsmaterials unter Verwendung des Lysepuffers nach Anspruch 1
➢ ggf. Vorfiltration, Bindung der Nukleinsäuren an eine feste Phase unter Verwendung des Bindungspuffers gemäß Anspruch 1 oder 2
➢ Waschen der gebundenen Nukleinsäuren
➢ Elution der gebundenen Nukleinsäuren.
   - ggf. Vorfiltration, Bindung der Nukleinsäuren an eine feste Phase
   - Waschen der gebundenen Nukleinsäuren
   - Elution der gebundenen Nukleinsäuren.

Gemäß einer bevorzugten Ausführungsform des erfindungsgemäßen Verfahrens wird zum Waschen der gebundenen Nukleinsäuren eine Mischung eines Puffers mit chaotropen und mit nicht-chaotropen Bestandteilen gemäß Anspruch 1 eingesetzt.

Das erfindungsgemäße Verfahren hat den Vorteil, dass für die Vorfiltration und die finale Adsorption der Nukleinsäuren dasselbe Filtermaterial verwendet wird.

Ebenfalls Gegenstand dieser Erfindung ist die Verwendung von Kombinationen aus Puffern mit chaotropen und mit nicht-chaotropen Bestandteilen zur Isolierung von Nukleinsäuren aus Nukleinsäuren enthaltenden Ausgangsmaterialien.

Erfindungsgemäß wurde ein Verfahren für die Isolierung und Aufreinigung von Nukleinsäuren aus komplexen Proben bereitgestellt, welches universell einsetzbar ist, unabhängig um welches Ausgangsmaterial es sich handelt und welches im Lysepuffer/Bindungspuffer ohne die bisher immer notwendigen hohen Salzkonzentrationen, die für eine Anbindung von Nukleinsäuren an Trägermaterialien notwendig sind, realisiert werden sollte. Darüber hinaus ermöglicht das Verfahren bei komplexen biologischen Proben (z.B. Gewebe) eine effiziente und schnelle Lyse des Ausgangsmaterials.

Es war davon auszugehen, das die Kombination von den ausführlich beschriebenen chaotropen Salzen und den nichtchaotropen bzw. antichaotropen Salzen als Bestandteile von Pufferformulierungen für die Isolierung von Nukleinsäuren aus komplexen biologischen Probe, keinen Effekt zeigen würde.

Überraschenderweise zeigte sich, dass die Verwendung von Lysepuffern zum Aufschluss einer Vielzahl ganz unterschiedlicher komplexer biologischer Proben (Zellen, Gewebe, Pflanzen, Bakterien etc.) auf der Basis von sehr geringen Konzentrationen an chaotropen Salzen in Kombination mit proteolytischen Enzymen (z.B. Proteinase K oder Lysozym) sowie einem Detergenz im Vergleich zu den bisher insbesondere kommerziell verfügbaren Lysepufferen als Kitkomponeten von Nukleinsäure-Reinigungs-Kits deutlich effizienter und schneller wirken. Nachteilig ist aber, dass die nur geringen chaotropen Salzkonzentrationen (< 100 mM) nachfolgend keine effiziente Anbindung der zu isolierenden Nukleinsäuren an dem Fachmann bekannte chromatographische Materialien ermöglichen und insbesondere auch die Reinheit der zu isolierenden Nukleinsäuren sehr gering ist. Kombiniert man allerdings den Lysepufferansatz, bestehend aus einem chaotropen Salz, einem Detergenz, einem proteolytischen Enzym u. ggf. weiteren Komponenten (Polyvinylpyrolidone, EDTA, Tris-HCl o.a.) mit einem Bindungspuffer, welcher eine nichtchaotrope Salzkomponente, einen Alkohol und ein Detergenz enthält und bringt diese Lösung nachfolgend in Kontakt mit einer nukleinsäurebindenden festen Phase (verwendet werden dem Fachmann an sich bekannte Materialien auf der Basis von Glas oder Silica), dann ist eine effiziente Isolierung und Aufreinigung von Nukleinsäuren möglich. Überraschenderweise sind die für die Anbindung der Nukleinsäuren notwendigen nichtchaotropen Salzkonzentrationen auch in viel geringeren Konzentrationen wirksam, als diese in den dem Fachmann bekanten Verfahren eingesetzt werden. So reichen Salzkonzentrationen von < 500 mM für eine effiziente Anbindung von zu isolierenden Nukleinsäuren aus. Der zu erwarten gewesene Effekt, dass sich die Wirkung diametral funktional entgegengesetzter Salze gegeneinander aufheben würde, tritt nicht ein.

Die erfindungsgemäße Kombination eines Lysepuffers mit einer chaotropen Salzkomponente und weiteren Zusätzen mit einem Bindungspuffer mit einer nichtchaotropen Salzkomponente verbindet in idealster Weise die Vorteile der beiden dem Fachmann bekannten Basistechnologien (chaotrope Chemie und antichaotrope Chemie). Mittels des beschriebenen Lysepuffers ist ein hocheffizienter und schneller Aufschluss des Ausgangsmaterials möglich. Darüber hinaus ermöglicht die chaotrope Komponente auch die Inaktivierung von Nukleinsäuren degradierender Enzyme während der Lyse des Ausgangsmaterials. Die nachfolgende Zugabe eines Bindungspuffers, welcher eine nichtchaotrope Salzkomponente enthält sowie weitere Zusätze, vermittelt nachfolgend eine hocheffiziente Anbindung von Nukleinsäuren an Trägermaterialien. Dabei bedeutet der nur sehr geringe Anteil der chaotropen Salzkomponente von kleiner 100 mM und der nur geringe Anteil an einer nichtchaotropen Salzkomponente von < 1 M, dass die für die Isolierung und Aufreinigung einzusetzenden Salzkonzentrationen deutlich geringer sind, als dem Fachmann bisher bekannt. Dies resultiert in einer möglichen Reduzierung von Waschschritten (und damit arbeitsaufwendigen Tätigkeiten) während der Nukleinsäureextraktion, reduziert die Kosten (insbesondere bei den Aufwendungen für chaotrope Salze), ist weniger gesundheitsgefährdend und reduziert auch das Problem der Verschleppung von chaotropen Salzen in downstream- Applikationen mit den bekannten starken inhibitorischen Wirkungen. Die Ausbeuten der zu isolierenden Nukleinsäuren sind dabei sehr hoch. Dies betrifft auch die Reinheit. Eine Kombination von chaotropen und nichtchaotropen Salzen für Verfahren zur Isolierung und Aufreinigung von Nukleinsäuren ist bisher nicht bekannt.

Nach der Lyse des Ausgangsmaterials und dem nachfolgenden Mischen mit dem beschriebenen Bindungspuffer wird die Probe mit einem Trägermaterial (z.B. einem Glasfaservlies als Bestandteil eines Zentrifugationsfilters) in Kontakt gebracht.

Die am Filtermaterial gebundene Nukleinsäure kann mit an sich bekannten Waschpuffern gewaschen, nachfolgend kurz getrocknet und nach Zugabe von Wasser bzw. 10mM Tris HCl oder anderen Niedrigsalzpuffern vom Glasfasermaterial wieder abgelöst werden. Überaschenderweise zeigte sich aber auch, dass die Verwendung von einem Waschpuffer, welcher sich aus Mischungen der erfindungsgemäßen Lysepuffer-Bindungspuffer zusammensetzt, sowohl die finale Ausbeute an Nukleinsäure als auch die Reinheit der isolierten Nukleinsäure noch verbessern lässt.

Da die im erfindungsgemäßen Verfahren eingesetzten Lysepuffer keinerlei Komponenten enthalten, die eine effiziente Adsorption von Nukleinsäuren ermöglichen, ist es auch möglich, z.B. notwendige Vorfilterprozesse über die selben Filtermaterialien zu realisieren, die auch final für die Adsorption der Nukleinsäuren eingesetzt werden. Dies stellt eine enorme verfahrenstechnologische Vereinfachung dar. So kann z.B. nach Lyse einer Pflanzenprobe das Lysat über eine Glasfaserfiltermatrix als Bestandteil einer Zentrifugationssäule zentrifugiert werden, um unlysierte Pflanzenmaterialien und inhibitorische Komponenten zu entfernen. Das Filtrat wird nachfolgend mit dem Bindungspuffer versetzt und auf eine weitere Zentrifugationssäule mit Glasfasermatrix überführt. Die Nukleinsäuren binden an die Fasern der Glasfasermatrix, werden mit einem alkoholhaltigen Waschpuffer gewaschen und die gebundenen Nukleinsäuren werden dann final von der Glasfasermatrix nach Zugabe von z.B. Wasser eluiert.

Dies gilt auch für solche komplexen biologischen Proben wie Stuhlproben oder u.U. auch Vollblut. Mit den bisher bekannten Systemen und Verfahren, welche im Lysepuffer auch die für die Adsorption der Nukleinsäuren notwendigen Salzkomponenten enthalten, kann eine solche Vereinfachung nicht realisiert werden.

Das erfindungsgemäße Verfahren zeigt überraschenderweise einen weiteren ganz

Das erfindungsgemäße Verfahren unterscheidet sich von dem Verfahren aus WO 2005/007895 A1 und WO 91/07422 A1 durch die besonders niedrige Konzentration eines chaotropen Bestandteils im Lysepuffer. Außerdem enthält der Bindungspuffer in dem o.g. Verfahren des Stands der Technik keinen Alkohol. Erst der Waschpuffer in WO 2005/007895 A1 enthält Alkohol. Zwar beschreibt WO 2005/007895 A1 eine relativ niedrige Konzentration von GuHCI im Lysepuffer, der die Protease enthält; stockt diese Konzentration aber vor Auftrag auf die Säule durch die Zugabe eines zweiten Chaotrops (500 µl 5 M GuSCN) auf.

wesentlichen neuen Effekt.

Es ist bekannt, dass die für die Isolierung und Aufreinigung eingesetzten Trägermaterialien (in Kombination mit den bekannten Hochsalzpuffern und ggf. Alkoholen) Glas, Keramik, Quarz, Silikagele, Aerosile, Diatomenerden etc. sind. Diese Materialien können porös und nichtporös sein. Sie können als Suspensionen oder auch als Fasern, Gele, Wolle oder Matten Bestandteile von z.B. Zentrifugationseinheiten (Zentrifugationsfiltersäulen) etc. sein. Dem Fachmann ist auch bekannt, dass die Bindung von Polyanionen, wie z.B. DNA an negative funktionale Oberflächen erfolgen kann. Dieses Basiswissen stellt den wissenschaftlichen Hintergrund für die Verwendung von negativen oder potentiell negativen festen Phasen für die Anbindung von Nukleinsäuren mit den bekannten Hochsalzpuffern dar.

Überraschenderweise zeigt die im erfindungsgemäßen Verfahren beschriebene Kombination eines Lysepuffers mit einer chaotropen Salzkomponente und einem Bindungspuffer mit einer nichtchaotropen Salzkomponente, dass eine effiziente Anbindung von Nukleinsäuren auch an eine Vielzahl von bisher nicht für die Isolierung und Aufreinigung von Nukleinsäuren eingesetzte Trägermaterialien möglich wird. Neben der Möglichkeit der Verwendung von an sich bekannten Trägermaterialien (insbesondere mit negativen funktionalen Ladungen) konnten physikalisch/chemisch gänzlich unterschiedliche Trägermaterialien für die Isolierung und Aufreinigung von Nukleinsäuren eingesetzt werden. Beispielhaft seien hierbei genannt:

positiv geladene Nylon-Membranen, Polysulfon-Membranen, Polyethersulfon-Membranen PVDF-Membranen, Membranen aus Acrylpolymeren, Ionenaustauscher-Membranen, Polyethylenfritten und selbst einfache Filterpapiere (z.B. Papierfilter). Dies ist insofern auch sehr überraschend, da eine Vielzahl der beschriebenen Membranen chemisch inerte, neutrale Oberflächen aufweisen, und eigentlich in der Praxis für Filtrationen eingesetzt werden, mit dem Ziel, keinerlei Bindungsaffinitäten gegenüber Biomolekülen zu haben.

Darüber hinaus eignen sich auch Partikel für die Anbindung der Nukleinsäuren mit dem beschriebenen Verfahren (z.B. funktionalisierte magnetische Eisenoxydpartikel, Silicatpartikel etc.).

Die Anbindung und die finale Desorption der Nukleinsäuren an und von diesen ganz unterschiedlichen Trägermaterialien kann dabei unter den gleichen Lyse/Bindungspufferbedingungen erfolgen.

Diese Beobachtung lässt auf einen völlig neuartigen Mechanismus schließen, über welchen sich der Prozess der Isolierung und Aufreinigung von Nukleinsäuren realisiert.

Es wird aber deutlich, dass die Möglichkeit des Einsatzes von den verschiedensten Trägermaterialien ganz neue Produktentwicklungen auf dem Gebiet der Isolierung und Aufreinigung von Nukleinsäuren ermöglichen wird. Die sinnvolle Kombination von Lyse/Bindungspuffer mit spezifischen Trägermaterialien bietet nunmehr die Möglichkeit völlig neuer Lösungsansätze für die Isolierung von Nukleinsäuren. Dies ist um so interessanter, da Methoden der molekularen Probenvorbereitung im Kontext der sich rasant entwickelnden molekularen Diagnostik in prinzipiell allen Bereichen unseres Lebens immer wesentlicher werden.

Die Erfindung soll nachfolgend anhand eines Ausführungsbeispiels näher erläutert werden. Das Beispiel stellt dabei keine Limitierung der Erfindung dar. Die Ausführungsbeispiele 2-4 dienen zum Nachweis, dass sich der erfinderische positive Effekt der Kombination von chaotropen und nichtchaotropen Puffern nicht nur auf die Kombination von 90mM Harnstoff im Lysepuffer und 250 mM ;Magnesiumchlorid im Bindungspuffer beschränkt.

### Ausführungsbeispiele

### Beispiel 1: Vergleich der Isolierung von genomischer DNA aus Gewebeproben unter Verwendung von Lysepuffer/Bindunspuffern auf der Basis nur chaotroper Salze, nur nichtchaotroper Salze und dem erfindungs gemäßen Verfahren

Jeweils 10 mg Gewebematerial (Schweineleber) wurden in 400 µl Lysepuffer A (4M Guanidinthiocyanat, 1% N-lauroyl-Sarcoisine, 2 mM EDTA), Lysepuffer B (1.5 M Ammoniumchlorid, 2 % CTAB, 10 mM Tris-HCl, 2 % PVP) sowie Lysepuffer C (0.5 % SDS, 10 mM Tris HCl, 2 mM EDTA, 90mM Harnstoff) in einem 1.5 ml Reaktionsgefäß unter Zugabe von 25 µl Proteinase K ( 20mg/ml ), sowie RNase A bei 50 °C unter kontinuierlichem Schütteln für 30 min inkubiert. Nach Lyse des Ausgangsmaterials wurde der Lyseansatz für 1 min bei Maximalgeschwindigkeit zentrifugiert um unlysierte Bestandteile abzutrennen. Der Überstand wurde nachfolgend wie folgt behandelt:
1. Lyseansatz mit Lysepuffer A direkt auf eine Spin Filter Säule mit Glasfasermaterial,
2. Zugabe von 200 µl eines Bindungspuffers (Triton X-100/ Isopropanol) zum Lyseansatz mit LysepufferB,
   danach Ansatz direkt auf eine Spin Filter Säule mit Glasfasemiaterial,
3. Zugabe von 400 µl eines Bindungspuffers (250 mM MgCl₂, Tween/Isopropanol) zum Lyseansatz mit Lysepuffer C, danach Ansatz direkt auf eine Spin Filter Säule mit Glasfasermaterial.

Nachfolgende Zentrifugation der Lösungen über die Glasfaserfilter.

Das Filtrat wurde danach verworfen und die Filtermaterialien bei den Varianten der. Lysepuffer A und Lysepuffer B sowie der Variante mit Lysepuffer C zweimal mit je 750 µl eines Waschpuffers (50 mM NaCl; 10 mM Tris HCl; 1 mM EDTA; 70% v/v Ethanol) gewaschen. In einer weiteren Extraktionsvariante mit dem erfindungsgemäßen Verfahren (Lysepuffer C) wurden die Filter einmal mit 500 µl aus einem Gemisch aus dem eingesetzten Lysepuffer C sowie dem dazugehörenden Bindungspuffer gewaschen und nachfolgend nochmals mit dem Standardwaschpuffer gewaschen.

Nach Ethanolentfernung durch einen kurzen Zentrifugationsschritt (12.000 rpm für 2min) erfolgte die Elution der Nukleinsäure durch Zugabe von 200µl eines Elutionspuffers (10 mM Tris-HCl; pH 8,5) durch Zentrifugation für 1 min bei 10.000 rpm. Nachfolgend erfolgte die spektrophotometrische Vermessung der DNA.

Die Ergebnisse sind in der nachfolgenden Tabelle dargestellt. Es wurden jeweils 3 Extraktionen durchgeführt und die Messwerte nach spektrophotometrischer Messung ermittelt und gemittelt.

| **Lysepuffer/Bindungspuffer** | **Ausbeute** | **Ratio A₂₆₀:A₂₈₀** |
|---|---|---|
| Chaotrop (Variante Lysepuffer A) | 8.2 µg | 1,4 |
| Nichtchaotrop (Variante Lysepuffer B) | 21,2 µg | 1,5 |
| Kombination chaotrop/nichtchaotrop (Variante Lysepuffer C) | 28,4 µg | 1,7 |
| Kombination chaotrop/nichtchaotrop (Variante Lysepuffer C) mit Waschpuffer aus einem Gemisch des eingesetzten Lysepuffers/Bindungspuffers | 36,2 µg | 1,8 |

Dem Fachmann ist ersichtlich, dass die verwendeten Lysepuffer A und B aus Patentschriften bekannt sind und beispielhaft für die beiden Basis-Technologien (chaotrope Chemie und nichtchaotrope Chemie) sind.

Es zeigt sich deutlich die Überlegenheit der erfindungsgemäßen Extraktion. Nach 30 min der Lysezeit, waren die Gewebeproben, welche mittels der erfindungsgemäßen Verfahrens behandelt wurden vollständig, die anderen Proben dagegen nur partiell lysiert.

Die Ergebnisse demonstrieren, dass die Isolierung von Nukleinsäuren mittels des erfindungsgemäßen Verfahrens als Kombination von chaotropen Salzen und nichtchaotropen Salzen (im Lysepuffer und Bindungspuffer) sehr effizient ist, die Lyse des Ausgangsmaterials deutlich schneller erfolgt und sowohl eine höhere Reinheit als auch höhere Ausbeuten realisiert werden können. Wird anstelle der bisher eingesetzten alkoholischen Standardwaschpuffer dagegen eine Mischung der erfindungsgemäßen Lyse/Bindungspuffer mit zum Waschen eingesetzt, dann führt diese Kombination noch zu einer Verbesserung des Extraktionserfolges.

### Beispiel 2: Kombination eines Lysepuffer, mit Bindungspuffern, welche neben Magnesiumchlorid weitere unterschiedliche nichtchaotrope Salze enthalten. Verwendung der Puffer entsprechend dem erfindungsgemäßen Verfahren zur Isolierung genomischer DNA aus einer komplexen biologischen Probe (Gewebe)

### Eingesetzte Lysepuffer und Bindungspuffer

### Lysepuffer: 90 mM Harnstoff/0.5% SDS

**Bindungspuffer H1:** 250 mM Magnesiumchlorid; 50% Isopropanol; 40 % Tween 20
**Bindungspuffer H2:** 250 mM Lithiumchlorid; 50% Isopropanol; 40 % Tween 20
**Bindungspuffer H3:** 250 mM Kaliumchlorid; 50% Isopropanol; 40 % Tween 20
**Bindungspuffer H4:** 250 mM Ammoniumchlorid; 50% Isopropanol; 40 % Tween 20

### Extraktionsprotokoll

1. Ca. 40 mg Lebergewebe (Maus) wurden je Ansatz mit 400 µl Lysepuffer und 25 µl Proteinase K versetzt und bei 50°C lysiert.
2. Nach Lyse erfolgte die Zugabe von je 400 µl Bindungspuffer (H1; H2; H3; H4). Lyseansatz und Bindungspuffer wurden mittels Pipette gründlich gemischt.
3. Überführen der Proben auf eine Zentrifugationssäule mit Glasfaserfiltermaterial und Zentrifugation bei 10.000 x g für 1 min. Verwerfen des Filtrates.
4. Nachfolgend zweimaliges Waschen mit einem ethanolhaltigen Waschpuffer (70% Ethanol, Natriumchlorid; Tris HCl).
5. Trocknung der Säule durch Zentrifugation für 2 min bei 10.000 x g.
6. Elution der DNA durch Zugabe von 200 µl eines Elutionspuffers (10 mM Tris HCl); Zentrifugation bei 5.000 x g für 1 min.

Anschließend wurde die isolierte DNA spektrophotometrisch vermessen. Die erhaltenen Messwerte sind Mittelwerte aus jeweils 3 Präparationen je verwendetem Bindungspuffer.

### Ergebnis

| Kombination Lysepuffer/Bindungspuffer | **Ratio A260:A280** | **Gesamtausbeute an DNA** |
|---|---|---|
| Lysepuffer: 90 mM Harnstoff/0.5% SDS | 2,0 | 68 µg |
| Bindungspuffer: H1 | | |
| Lysepuffer: 90 mM Harnstoff/0.5% SDS | 1,95 | 69 µg |
| Bindungspuffer: H2 | | |
| Lysepuffer: 90 mM Harnstoff/0.5% SDS | 1,95 | 65 µg |
| Bindungspuffer: H3 | | |
| Lysepuffer: 90 mM Harnstoff/0.5% SDS | 1,96 | 64 µg |
| Bindungspuffer: H4 | | |

### Schussfolgerung:

Die Kombination des vorliegenden Lysepuffers mit unterschiedlichen Bindungspuffern (Unterscheidung der Bindungspuffer in den eingesetzten nichtchaotropen Salzen) führt zu dem Ergebnis, dass die verwendeten nichtchaotropen Salze alle hervorragend für die Isolierung der Nukleinsäuren geeignet sind. Damit zeigt dieses Ergebnis auch, dass nicht nur Magnesiumchlorid den erfindungsgemäßen positiven Effekt der Kombination eines Lysepuffers mit einem chaotropen Salz und einem Bindungspuffer mit einem nichtchaotropen Salz bewirkt, sondern andere nichtchaotrope Salze (.B. Lithiumchlorid; Kaliumchlorid; Ammoniumchlorid) den erfindungsgemäßen Effekt ebenso stützen und als nichtchaotrope Salze in Bindungspuffern ebenso verwendet werden können.

### Beispiel 3: Verwendung von Lysepuffern, die sich im eingesetzten chaotropen Salz unterscheiden und Kombination dieser Lysepuffer mit zwei verschiedenen Bindungspuffern, um zu beweisen, dass sich die Erfindung nicht nur auf die Nutzung von Harnstoff als chaotropem Salz im Lysepuffer bezieht. Eingesetzt wurden die beiden chaotropen Salze Harnstoff und Natriumperchlorat.

### Eingesetzte Lysepuffer und Bindungspuffer

**Lysepuffer A:** 90 mM Harnstoff/0.5% SDS
**Lysepuffer B:** 90 mM Natriumperchlorat/0.5%

**Bindungspuffer H1:** 250 mM Magnesiumchlorid; 50% Isopropanol; 40 % Tween 20
**Bindungspuffer H3:** 250 mM Kaliumchlorid; 50% Isopropanol; 40 % Tween 20

### Extraktionsprotokoll

1. Ca. 25 mg Lebergewebe (Maus) wurden je Ansatz mit 400 µl Lysepuffer (Lysepuffer A ; Lysepuffer B) und 25 µl Proteinase K versetzt und bei 50°C lysiert.
2. Nach Lyse erfolgte die Zugabe von je 400 µl Bindungspuffer (H1; H3). Lyseansatz und Bindungspuffer wurden mittels Pipette gründlich gemischt.
3. Überführen der Proben auf eine Zentrifugationssäule mit Glasfaserfiltermaterial und Zentrifugation bei 10.000 x g für 1 min. Verwerfen des Filtrates.
4. Nachfolgend zweimaliges Waschen mit einem ethanolhaltigen Waschpuffer (70% Ethanol, Natriumchlorid; Tris HCl).
5. Trocknung der Säule durch Zentrifugation für 2 min bei 10.000 x g.
6. Elution der DNA durch Zugabe von 200 µl eines Elutionspuffers (10 mM Tris HCl); Zentrifugation bei 5.000 x g für 1 min.

Anschließend wurde die isolierte DNA spektrophotometrisch vermessen. Die erhaltenen Messwerte sind Mittelwerte aus jeweils 3 Präparationen je verwendetem Bindungspuffer.

### Ergebnis

| Kombination Lysepuffer/Bindungspuffer | **Ratio A260:A280** | **Gesamtausbeute an DNA** |
|---|---|---|
| Lysepuffer: 90 mM Harnstoff/0.5% SDS | 1,96 | 41 µg |
| Bindungspuffer: H1 | | |
| Lysepuffer: 90 mM Hamstoff/0.5% SDS | 1,98 | 39 µg |
| Bindungspuffer: H3 | | |
| Lysepuffer: 90 mM Natriumperchlorat/0.5% SDS | 1,94 | 37 µg |
| Bindungspuffer: H1 | | |
| Lysepuffer: 90 mM Natriumperchlorat/0.5% SDS | 1,98 | 41 µg |
| Bindungspuffer: H3 | | |

### Schussfolgerung:

Die beiden eingesetzten Lysepuffer unterscheiden sich im verwendeten chaotropen Salz. Beide Lysepuffer realisieren hervorragend die Isolierung der genomischen DNA, wenn sie in Kombination mit Bindungspuffern ,die nichtchaotrope Salze enthalten, eingesetzt werden. Dabei können auch in diesem Beispiel andere nichtchaotrope Salze als Magnesiumchlorid als Bestandteil der Bindungspuffer zum Einsatz kommen. Die erfindungsgemäßen Vorteile beschränken sich nicht nur auf die Verwendung von Harnstoff im Bindungspuffer.

### Beispiel 4: Kombination eines Lysepuffers mit enthaltenem chaotropen Salz und Bindungspuffern, welche nichtchaotrope Salze enthalten, wobei die Ionenstärke der im Bindungspuffer zum Einsatz kommenden nichtchaotropen Salze deutlich reduziert wurde. Der Versuch soll unterstreichen, dass die im erfindungsgemäßen Verfahren dargestellte Kombination von chaotropen und nichtchaotropen Salzen in Lysepuffer und Bindungspuffer die Isolierung von Nukleinsäuren auch mit extrem geringen Salzkonzentrationen erlaubt.

### Eingesetzte Lysepuffer und Bindungspuffer

**Lysepuffer:** 90 mM Harnstoff/0.5% SDS
**Bindungspuffer H5:** 100 mM Magnesiumchlorid; 50% Isopropanol; 40 % Tween 20
**Bindungspuffer H6:** 100 mM Lithiumchlorid; 50% Isopropanol; 40 % Tween 20
**Bindungspuffer H7:** 100 mM Kaliumchlorid; 50% Isopropanol; 40 % Tween 20

### Extraktionsprotokoll

1. Ca. 25 mg Lebergewebe (Maus) wurden je Ansatz mit 400 µl Lysepuffer und 25 ul Proteinase K versetzt und bei 50°C lysiert.
2. Nach Lyse erfolgte die Zugabe von je 400 µl Bindungspuffer (H5; H6; H7). Lyseansatz und Bindungspuffer wurden mittels Pipette gründlich gemischt.
3. Überführen der Proben auf eine Zentrifugationssäule mit Glasfaserfiltermateriat und Zentrifugation bei 10.000 x g für 1 min. Verwerfen des Filtrates.
4. Nachfolgend nur einmaliges Waschen mit einem ethanolhaltigen Waschpuffer (70% Ethanol, Natriumchlorid; Tris HCl).
5. Trocknung der Säule durch Zentrifugation für 2 min bei 10.000 x g.
6. Elution der DNA durch Zugabe von 200 µl eines Elutionspuffers (10 mM Tris HCl); Zentrifugation bei 5.000 x g für 1 min.

Anschließend wurde die isolierte DNA spektrophotometrisch vermessen. Die erhaltenen Messwerte sind Mittelwerte aus jeweils 3 Präparationen je verwendetem Bindungspuffer.

### Ergebnis

| Kombination Lysepuffer/Bindungspuffer | **Ratio A260:A280** | **Gesamtausbeute an DNA** |
|---|---|---|
| Lysepuffer: 90 mM Harnstoff/0.5% SDS | 1,73 | 43 µg |
| Bindungspuffer: H5 | | |
| Lysepuffer: 90 mM Harnstoff/0.5% SDS | 1,80 | 42 µg |
| Bindungspuffer: H6 | | |
| Lysepuffer: 90 mM Harnstoff/0.5% SDS | 1,71 | 44 µg |
| Bindungspuffer: H7 | | |

### Schussfolgerung:

Die Kombination des vorliegenden Lysepuffers mit unterschiedlichen Bindungspuffern (Unterscheidung der Bindungspuffer in den eingesetzten nichtchaotropen Salzen) führt zu dem Ergebnis, dass die verwendeten nichtchaotropen Salze alle hervorragend für die Isolierung der Nukleinsäuren geeignet sind. Damit zeigt dieses Ergebnis auch, dass nicht nur Magnesiumchlorid den erfindungsgemäßen positiven Effekte der Kombination eines Lysepuffers mit einem chaotropen Salz und einem Bindungspuffer mit einem nichtchaotropen Salz bewirkt, sondern andere nichtchaotrope Salze ebenso verwendet werden können. Darüber hinaus verdeutlicht das Ergebnis eindrucksvoll, dass gemäß dem erfindungsgemäßen Verfahren sehr viel geringere Ionenstärken für die Isolierung von Nukleinsäuren über das dem Fachmann bekannte Prinzip der Bindung von Nukleinsäuren an feste mineralische Materialien notwendig sind, als dies nach dem Stand der Technik bisher bekannt ist. Damit erlaubt die Kombination der erfindungsgemäßen Lysepuffer/Bindungspuffer eine extrem effiziente Isolierung von Nukleinsäuren ohne die bisher notwendigen hohen Salzkonzentrationen in den eingesetzten Puffersystemen. Dies ermöglicht es auch, wie im beschriebenen Versuch, die notwendigen Waschschritte zu reduzieren.

### Definitionen

### Chaotrope Bestandteile:

Substanzen, die regelmäßige - auf der Bildung von Wasserstoffbrückenbindungen beruhende - Struktur von flüssigem Wasser zerstören, indem sie die Bildung der zur Solvatation notwendigen H₂O-Käfigstrukturen verhindern. Beispiele für chaotrope Bestandteile sind Thiocyanate, Iodide oder Perchlorate.

### Antichaotrope Bestandteile:

Substanzen, die regelmäßige - auf der Bildung von Wasserstoffbrückenbindungen beruhende - Struktur von flüssigem Wasser verstärken. Beispiele für anti-chaotrope Bestandteile sind Ammonium-, Natrium- oder Kaliumsalze.

### Alkoholische Komponente:

Alkoholische Komponenten im Sinne der Erfindung sind alle wasserlöslichen Alkohole, wie Methanol, Ethanol, Propanol, Isopropanol, Ethylenglycol, Polyethylenglycol oder Gycerin.

## Patentansprüche

1. Formulierungen zur Isolierung von Nukleinsäuren aus Nukleinsäuren enthaltenden Materialien, umfassend
• mindestens einen Lysepuffer mit chaotropen Salzkomponenten, der zusätzlich mindestens ein proteolytisches Enzym und mindestens ein Detergenz enthält und
• mindestens einen Bindungspuffer mit nicht-chaotropen Salzkomponenten, der zusätzlich mindestens einen Alkohol und mindestens ein Detergenz enthält,
**dadurch gekennzeichnet, dass** die Konzentration der chaotropen Salzkomponenten des Lysepuffers kleiner als 100mM und die Konzentration der nicht-chaotropen Salzkomponenten des Bindungspuffers kleiner als 1M ist.

2. Formulierungen nach Anspruch 1, **dadurch gekennzeichnet, dass** die Konzentration der nicht-chaotropen Salzkomponenten des Bindungspuffers kleiner als 500mM ist.

3. Formulierungen nach Anspruch 1, **dadurch gekennzeichnet, dass** die alkoholische Komponente aus wasserlöslichen Alkoholen, wie Methanol, Ethanol, Propanol, Isopropanol, Ethylenglycol, Polyethylenglycol oder Gycerin besteht.

4. Formulierungen nach Anspruch 3, **dadurch gekennzeichnet, dass** der Anteil der alkoholischen Komponente 20 bis 80%, vorzugsweise 45 bis 55 % beträgt.

5. Formulierungen nach Anspruch 1, **dadurch gekennzeichnet, dass** als Detergenzkomponente mindestens eines der Substanzen Polyvinylpyrrolidin, CTAB, TritonX-100, N-Lauryl-Sarkosin, Natriumcitrat, DDT oder Tween 20 eingesetzt werden.

6. Formulierungen nach Anspruch 1, **dadurch gekennzeichnet, dass** sie zusätzlich eine feste Phase umfassen.

7. Formulierungen nach Anspruch 6, **dadurch gekennzeichnet, dass** als feste Phase positiv geladene Nylon-Membranen, Polysulfon-Membranen, Polyethersulfon-Membranen PVDF-Membranen, Membranen aus Acrylpolymeren, Ionenaustauscher-Membranen, Polyethylenfritten, einfache Filterpapiere, magnetische Eisenoxydpartikel oder Silicatpartikel eingesetzt werden.

8. Verfahren zur Isolierung von Nukleinsäuren aus Nukleinsäuren enthaltenden Materialien unter Verwendung von Formulierungen nach einem der Ansprüche 1 bis 7, umfassend folgende Schritte:
➢ Lyse des Ausgangsmaterials unter Verwendung des Lysepuffers nach Anspruch 1
➢ ggf. Vorfiltration,
➢ Bindung der Nukleinsäuren an eine feste Phase unter Verwendung des Bindungspuffers gemäß Anspruch 1 oder 2
➢ Waschen der gebundenen Nukleinsäuren
➢ Elution der gebundenen Nukleinsäuren.

9. Verfahren nach Anspruch 8, **dadurch gekennzeichnet, dass**, dass zum Waschen der gebundenen Nukleinsäuren eine Mischung eines Puffers mit chaotropen und mit nicht-chaotropen Salzkomponenten gemäß Anspruch 1 eingesetzt wird.

10. Verfahren nach Anspruch 9, **dadurch gekennzeichnet, dass** für die Vorfiltration und die finale Adsorption der Nukleinsäuren dasselbe Filtermaterial verwendet wird.

## Claims

1. Formulations for isolating nucleic acids from materials containing nucleic acids, comprising
• at least a lysis buffer with chaotropic salt components which contains in addition at least a proteolytic enzyme and at least a detergent, and
• at least a bonding buffer with non-chaotropic salt components which contains in addition at least an alcohol and at least a detergent,
**characterised in that** the concentration of the chaotropic salt components of the lysis buffer is smaller than 100mM, and the concentration of the non-chaotropic salt components of the bonding buffer is smaller than 1M.

2. Formulations according to claim 1, **characterised in that** the concentration of the non-chaotropic salt components of the bonding buffer is smaller than 500mM.

3. Formulations according to claim 1, **characterised in that** the alcoholic component comprises a water-soluble alcohol such as methanol, ethanol, propanol, isopropanol, ethylene glycol, polyethylene glycol or glycerin.

4. Formulations according to claim 3, **characterised in that** the proportion of the alcoholic component is 20 % to 80 %, preferably 45 to 55 %.

5. Formulations according to claim 1, **characterised in that** as a detergent component at least one of the substances of polyvinylpyrrolidine, CTAB, TritonX-100, N-Lauryl-sarkosin, sodium citrate, DDT or Tween 20 are used.

6. Formulations according to claim 1, **characterised in that** they comprise in addition a solid phase.

7. Formulations according to claim 6, **characterised in that** positively charged nylon membranes, polysulfone membranes, polyethersulfone membranes, PVDF membranes, membranes from acrylic polymers, ion exchange membranes, polyethylene frit membranes, simple filter papers, magnetic iron oxide particles or silicate particles are used as a solid phase.

8. Method for isolating nucleic acids from materials containing nucleic acids by using form-ulations according to any one of claims 1 to 7, comprising the following steps:
➢ lysis of the starting material by using the lysis buffer according to claim 1
➢ prefiltration, if required
➢ bonding the nucleic acids to a solid phase by using the bonding buffer according to claim 1 or 2
➢ washing of the bonded nucleic acids
➢ eluting of the bonded nucleic acids.

9. Method according to claim 8, **characterised in that** for washing of the bonded nucleic acids a mixture of a buffer of chaotropic and non-chaotropic salt components according to claim 1 is used.

10. Method according to claim 9, **characterised in that** for prefiltration and final adsorption of the nucleic acids the same filter material is used.

## Revendications

1. Formules pour isolation des acides nucléiques à partir des matériaux contenant des acides nucléiques et comprenant:
• au moins un tampon de lyse avec des composants de sel chaotrope comportant en outre au moins une enzyme protéolytique et au moins un détergent et
• au moins un tampon de liaison avec des composants de sel non chaotrope comportant en outre au moins un alcool et au moins un détergent,
**caractérisé en ce que** la concentration des composants de sel chaotrope du tampon de lyse est inférieure à 100mM et la concentration des composants de sel non chaotrope du tampon de liaison est inférieure à 1M.

2. Formules selon la revendication 1, **caractérisé en ce que** la concentration des composants de sel non chaotrope du tampon de liaison est inférieure à 500mM.

3. Formules selon la revendication 1, **caractérisé en ce que** le composant alcoolique comporte des alcools solubles dans l'eau comme du méthanol, d'éthanol, du propanol, d'isopropanol, d'éthylène glycol, du polyéthylène glycol ou du glycérine.

4. Formules selon la revendication 3, **caractérisé en ce que** la part du composant alcoolique est 20 % à 80 %, de préférence 45 à 55%.

5. Formules selon la revendication 1, **caractérisé en ce qu'**au moins une des substances de polyvinyle de pyrrolidine, CTAB, TritonX-100, N-Lauroylsarcosine, citrate de sodium, DDT ou Tween 20 sont utilisées en tant que composant de détergent.

6. Formules selon la revendication 1, **caractérisé en ce qu'**elles comprend en outre une phase solide.

7. Formules selon la revendication 6, **caractérisé en ce que** des membranes de nylon chargées positivement, des membranes de polysulfone, des membranes de polyéther sulfones, des membranes PVDF, des membranes en polymère acrylique, des membranes d'échangeur d'ions, des frittes en polyéthylène, des simples papiers filtres, des particules magnétiques en oxyde de fer ou des particules en silicate sont utilisés en tant que phase solide.

8. Procédé d'isolation des acides nucléiques à partir des matériaux contenant des acides nucléiques en utilisant des formules selon l'une quelconque des revendications 1 à 7, comportant les étapes suivantes:
➢ lyse du matériau de base en utilisant le tampon de lyse selon la revendication 1
➢ préfiltration, le cas échéant
➢ liaison des acides nucléiques à une phase solide en utilisant le tampon de liaison selon la revendication 1 ou 2
➢ lavage des acides nucléiques liés
➢ élution des acides nucléiques liés.

9. Procédé selon la revendication 8, **caractérisé en ce que** pour le lavage des acides nucléiques liés un mélange d'un tampon avec des composants de sel chaotrope et avec des composants de sel non chaotrope selon la revendication 1 est utilisé.

10. Procédé selon la revendication 9, **caractérisé en ce que** pour la préfiltration et l'adsorption finale des acides nucléiques le même matériel de filtre est utilisé.
